# EUROPEAN PATENT APPLICATION

(11) **EP 4 512 309 A1**
(43) Date of publication of application: **26.02.2025**
(21) Application number: 23792146.5
(22) Date of filing: 18.04.2023
(51) Int. Cl.: A61B 5/00, G16H 10/20, G16H 50/20, G16H 50/30

(54) **DEVICE AND METHOD FOR PROVIDING SKIN INFORMATION ON BASIS OF SKIN MICROBIOME**

(30) Priority: 19.04.2022 KR 20220048315
(71) Applicant: Cosmax, Inc., Hwaseong-si, Gyeonggi-do 18622 (KR)
(72) Inventor: LEE, Dong Geol, Hwaseong-si, Gyeonggi-do 18496 (KR); JO, Hyung Woo, Seongnam-si, Gyeonggi-do 13486 (KR); HEO, Young Mok, Seongnam-si, Gyeonggi-do 13486 (KR); BAEK, Chae Yun, Seongnam-si, Gyeonggi-do 13486 (KR); KIM, Hye Been, Seongnam-si, Gyeonggi-do 13486 (KR); KANG, Seung Hyun, Seoul 06285 (KR); PARK, Myeong Sam, Seongnam-si, Gyeonggi-do 13486 (KR)
(74) Representative: Lavoix
(86) International application number: PCT/KR2023/005226
(87) International publication number: WO 2023/204570

(57) **Abstract**

The present disclosure provides a device and method for providing skin information based on the skin microbiome. The device for providing skin information includes a communication unit forming a network connection with an external device, a user interface unit, a processor, and a memory storing instructions executable by the processor, wherein the processor is configured to, by executing the instructions, obtain survey information related to a user's skin condition and, based on the survey information, select the user's skin type, analyze the user's skin microbiome distribution, or provide information about the user's skin.

## Description

### Technical Field

Embodiments of the present disclosure relate to a device and method for providing skin information based on the skin microbiome.

### Background Art

Recently, with the growth of the cosmetics market and the development of communication technology, a vast amount of information related to cosmetic products has been accumulated on the Internet. When people want to purchase cosmetic products, many of them refer to reviews posted by other people on the Internet or search for information related to purchasing cosmetic products posted by cosmetic product sales companies, such as the brand name, price, product appearance, packaging, usage instructions, and ingredients used in manufacturing the cosmetic products to determine the cosmetics they want. However, in the cosmetic product market, there are various brand names, numerous product types, and similar products, so the market itself is very large and the types of cosmetic products and information about them are also diverse. Because of this, consumers of cosmetic products spend time sorting from through a vast amount of information to choose which cosmetic product to purchase. Therefore, there is an emerging need to provide customized information to consumers of cosmetic products.

### Disclosure

### Technical Problem

The present disclosure is intended to solve various problems including the problems described above, and aims to provide a device and method for providing skin information based on the skin microbiome. However, this objective is illustrative and does not limit the scope of the present disclosure.

### Technical Solution

According to an aspect of the present disclosure, a device for providing skin information includes a communication unit forming a network connection with an external device, a user interface unit, a processor, and a memory storing instructions executable by the processor, wherein the processor is configured to, by executing the instructions, obtain survey information related to a user's skin condition and, based on the survey information, select the user's skin type, analyze the user's skin microbiome distribution, or provide information about the user's skin.

The processor may be further configured to, by executing the instructions, obtain subjective survey information including the user's subjective response in relation to the user's skin condition, and objective survey information including clinical data, microbiome data, human genome data, or metabolite data in relation to the user's skin condition.

The processor may be further configured to, by executing the instructions, derive key skin factors based on the subjective survey information and the objective survey information, the key skin factors corresponding to the user's skin condition and being used in a skin type selection algorithm and a skin microbiome analysis algorithm.

The processor may be further configured to, by executing the instructions, distinguish skin types according to oil and moisture and color and elasticity and select the user's skin type by using analysis information of the survey information about key skin factors.

The key skin factors may include elasticity, skin tone, oil amount, moisture loss amount, nasolabial folds, or pores.

The processor may be further configured to, by executing the instructions, derive information about a plurality of microbiomes corresponding to the user's skin condition with respect to a skin microbiome distribution analyzed by age.

The processor may be further configured to, by executing the instructions, provide information about a mixing ratio of each microbiome with respect to a plurality of microbiomes corresponding to the user's skin condition.

The processor may be further configured to, by executing the instructions, select the user's skin type by using a pre-learned skin type selection algorithm.

The processor may be further configured to, by executing the instructions, analyze the user's skin microbiome distribution by using a pre-learned skin microbiome analysis algorithm based on the survey information.

The processor may be further configured to, by executing the instructions, provide information about the user's skin based on the user's skin type or the user's skin microbiome distribution.

According to an aspect of the present disclosure, a method of providing skin information includes obtaining survey information related to a user's skin condition, and selecting the user's skin type by using a pre-learned skin type selection algorithm based on the survey information, analyzing the user's skin microbiome distribution by using a pre-learned skin microbiome analysis algorithm based on the survey information, or providing information about the user's skin based on the survey information.

The obtaining of the survey information may include obtaining subjective survey information including the user's subjective response in relation to the user's skin condition, and objective survey information including clinical data, microbiome data, human genome data, or metabolite data in relation to the user's skin condition.

The obtaining of the survey information may include deriving key skin factors based on the subjective survey information and the objective survey information, the key skin factors corresponding to the user's skin condition and being used in the skin type selection algorithm and the skin microbiome analysis algorithm.

The selecting of the user's skin type may include distinguishing skin types according to oil and moisture and color and elasticity and selecting the user's skin type, by using analysis information of the survey information about key skin factors.

The key skin factors may include elasticity, skin tone, oil amount, moisture loss amount, nasolabial folds, or pores.

The analyzing of the user's skin microbiome distribution may include deriving information about a plurality of microbiomes corresponding to the user's skin condition with respect to a skin microbiome distribution analyzed by age.

The providing of the information about the user's skin may include providing information about a mixing ratio of each microbiome with respect to a plurality of microbiomes corresponding to the user's skin condition.

The providing of the information about the user's skin may include providing information about the user's skin based on the user's skin type or the user's skin microbiome distribution.

According to an aspect of the present disclosure, a computer program stored in a computer-readable recording medium to execute the method by using a computing device is provided.

Other aspects, features, and advantages other than those described above will become clear from the detailed description, claims, and drawings for carrying out the disclosure below.

### Advantageous Effects

According to an embodiment of the present disclosure as described above, a device and method for providing skin information, which may effectively provide optimized skin information to a user based on the skin microbiome, may be implemented.

In addition, according to the present disclosure, it is possible to provide an immersive and empathetic solution by analyzing exact skin conditions and potential skin factors. However, the scope of the present disclosure is not limited by this effect.

### Description of Drawings

FIG. 1 is a conceptual diagram schematically showing a skin information providing device according to an embodiment of the present disclosure and a server.
FIG. 2 is a block diagram schematically showing the components of a skin information providing device according to an embodiment of the present disclosure.
FIG. 3 is a flowchart showing a method of providing skin information, according to an embodiment of the present disclosure.
FIG. 4 is a diagram for explaining a method of providing skin information, according to an embodiment of the present disclosure.
FIGS. 5 to 8 show examples of survey information related to a user's skin condition according to an embodiment of the present disclosure.
FIG. 9 shows an example of key factors corresponding to a user's skin condition according to an embodiment of the present disclosure.
FIGS. 10A to 10D are diagrams for explaining a skin type selection process according to an embodiment of the present disclosure.
FIG. 11 is a diagram for explaining a skin type selection process according to another embodiment of the present disclosure.
FIG. 12 is a diagram for explaining a microbiome analysis process according to an embodiment of the present disclosure.
FIG. 13 is a diagram for explaining a method of providing skin information, according to another embodiment of the present disclosure.

### Mode for Invention

Because various changes can be applied to the present disclosure and the present disclosure can have various embodiments, particular embodiments are illustrated in the drawings and described in detail. Effects and features of the present disclosure and methods of securing them may be apparent with reference to the embodiments described below in detail with reference to the drawings. However, the present disclosure is not limited to the embodiments disclosed below, but may be implemented in various forms.

Hereinafter, embodiments of the present disclosure are described in detail with reference to the accompanying drawings, and when described with reference to the drawings, the same or corresponding components are given the same reference numerals, and duplicate descriptions thereof are omitted.

It will be understood that although the terms "first," "second," etc. may be used herein to describe various components, these components should not be limited by these terms. These components are only used to distinguish one component from another. In addition, singular expressions include plural expressions unless they are explicitly and differently specified in context. In addition, it will be further understood that the terms "includes or comprises" and/or "including or comprising" used in the present disclosure specify the presence of stated features or components, but do not preclude the presence or addition of one or more other components or features.

Sizes of components in the drawings may be exaggerated or reduced for convenience of explanation. For example, because sizes and thicknesses of components in the drawings are arbitrarily illustrated for convenience of explanation, the present disclosure is not limited thereto.

In the following embodiments, when a part such as a region, component, portion, block, or module is said to be above or on another part, this case includes not only the case where the part is directly on the other part, but also the case where there are another region, component, portion, block, or module in between. In addition, when regions, components, portions, blocks, or modules are connected to each other, this case includes the case where the regions, components, portions, blocks, or modules are directly connected to each other, but also the case where the regions, components, portions, blocks, or modules are indirectly connected with another region, component, portion, block, or module interposed therebetween.

A " device for providing skin information (hereinafter, referred to as a skin information providing device)" may refer to a device used to provide skin information. A program used to provide skin information may be installed in the skin information providing device. For example, the skin information providing device may perform at least one operation of providing skin information based on information input from a program through a server. For example, the skin information providing device may be a mobile terminal device, such as a smartphone, as a user terminal provided by the user.

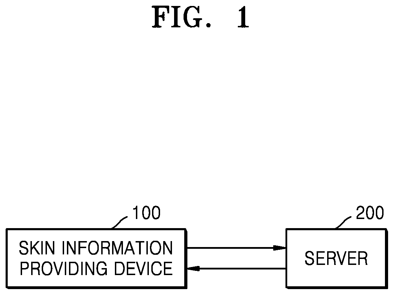

FIG. 1 is a conceptual diagram schematically showing a skin information providing device 100 according to an embodiment of the present disclosure and a server 200.

Referring to FIG. 1, the skin information providing device 100 may be a device connected to the server 200 and a network. In FIG. 1, one server and one skin information providing device are connected to the network. However, as an example, a plurality of servers and/or a plurality of skin information providing devices may be connected to the network. The server 200 may be, for example, a cloud server, but the present disclosure is not limited thereto.

The network may be defined as one or more data links that may transmit and receive data between electronic devices and/or servers, and may be a wired and/or wireless communication network.

The skin information providing device 100 may obtain survey information related to the user's skin condition and may select the user's skin type, analyze the user's skin microbiome distribution, or provide information about the user's skin, based on the survey information.

The skin information providing device 100 may obtain survey information related to the user's skin condition, select the user's skin type by using a pre-learned skin type selection algorithm based on the survey information, analyze the user's skin microbiome distribution by using a pre-learned skin microbiome analysis algorithm based on the survey information, and provide information about the user's skin based on the user's skin type and the user's skin microbiome distribution.

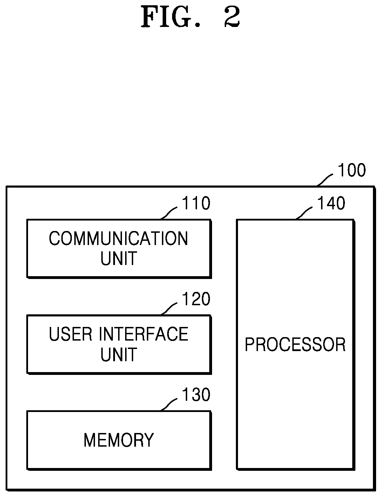

FIG. 2 is a block diagram schematically showing the components of the skin information providing device according to an embodiment of the present disclosure.

Referring to FIG. 2, the skin information providing device 100 according to an embodiment of the present disclosure may include a communication unit 110, a user interface unit 120, a memory 130, and a processor 140. Below, the components are described.

The communication unit 110 is not particularly limited and may include a communication module supporting one of various communication methods. For example, the communication module may be in the form of a chipset, or may be a sticker/barcode (e.g. a sticker including a near field communication (NFC) tag) including information necessary for communication. In addition, the communication module may be a short-distance communication module or a wired communication module. In addition, not only a communication method utilizing a communication network (for example, mobile communication network, wired Internet, wireless Internet, broadcasting network, or satellite network) that includes or may be connected to a network, but also short-distance wireless communication between loT devices may be included in the communication module.

For example, the communication unit 110 may support at least one of Wireless LAN, Wireless Fidelity (Wi-Fi), Wi-Fi Direct (WFD), Bluetooth, Bluetooth Low Energy (BLE), Wired Lan, Near Field Communication (NFC), Zigbee, Infrared Data Association (IrDA), 3G, 4G, and 5G, but is not limited thereto.

The user interface unit 120 may include an input unit for receiving, from the user, an input for controlling the operation of the skin information providing device 100, and an output unit for displaying information, such as the results of the operation of the skin information providing device 100 or the status of the skin information providing device 100. For example, the user interface unit 120 may include a manipulation panel that receives a user input, a display panel that displays a screen, and the like.

Specifically, the input unit may include devices that may receive various types of user input, for example, a keyboard, physical button, touch screen, camera, or microphone. In addition, the output unit may include, for example, a display panel or a speaker. However, the user interface unit 120 is not limited thereto and may include a device that supports various inputs and outputs.

The memory 130 may include or be any non-transitory readable computer recording medium or storage device. According to an embodiment, the memory 130 may include a permanent mass storage device, such as read only memory (ROM), a disk drive, solid state drive (SSD), or flash memory. As another example, the permanent mass storage device, such as ROM, SSD, flash memory, a disk drive, may be included in a separate information processing system as a separate permanent storage device distinct from memory. In addition, an operating system and at least one program code (e.g., code for an unattended storage service) may be stored in the memory 130. Furthermore, the memory 130 may include not only a single memory but also a plurality of memories.

The memory 130 may store instructions, software, or programs. Hereinafter, the software or programs may refer to software or programs used by electronic devices, such as the skin information providing device 100 and the server 200. For example, the memory 130 may store instructions for how to operate the skin information providing device 100. The instructions, software, or programs may be stored in a database (DB) format, but are not limited thereto.

The processor 140 may control all operations of the skin information providing device 100 and may include at least one processor, such as central processing unit (CPU). The processor may include at least one processor specialized for each function, or may be an integrated processor. For example, the processor may include a skin information providing module that performs a skin information providing operation. For example, the processor may call at least one application programming interface (API) used to perform a skin information providing operation.

In addition, in other embodiments, the skin information providing device 100 may include more components than those shown in FIG. 2. For example, the skin information providing device 100 may be implemented to include an input/output device or may further include other components, such as a battery and charging device that supplies power to internal components, various sensors, and a database.

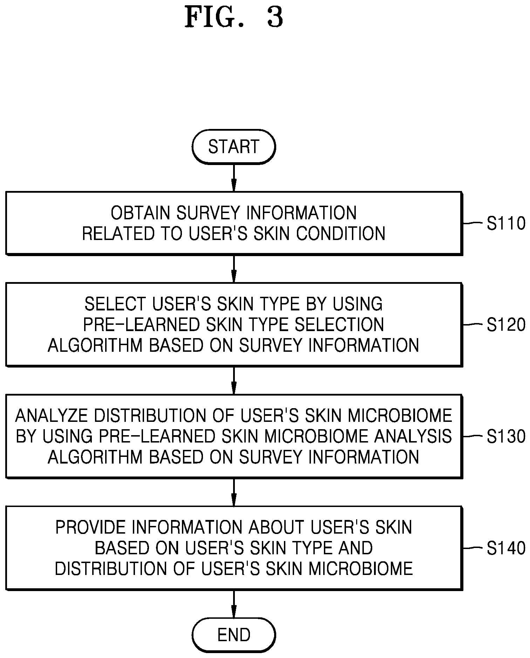

The processor 140 and the components of the processor 140 may be implemented to execute instructions according to the code of an operating system included in the memory 130 and the code of at least one program. Here, the components of the processor 140 may be expressions of different functions of the processor 140, which are performed by the processor 140 according to instructions provided by the program code stored in the server 200. The specific operation of the processor 140 will be described with reference to the flowchart of a method of providing skin information in FIG. 3.

FIG. 3 is a flowchart showing a method of providing skin information, according to an embodiment of the present disclosure.

In operation S110, the skin information providing device 100 may obtain survey information related to the user's skin condition.

The skin information providing device 100 according to an embodiment of the present disclosure may obtain subjective survey information including the user's subjective response in relation to the user's skin condition. In addition, the skin information providing device 100 may obtain objective survey information including clinical data, microbiome data, human genome data, and/or metabolite data in relation to the user's skin condition.

In addition, the skin information providing device 100 according to an embodiment of the present disclosure may derive key factors corresponding to the user's skin condition based on the subjective survey information and the objective survey information. Here, the key factors may be used in a skin type selection algorithm and a skin microbiome analysis algorithm.

In operation S120, the skin information providing device 100 may select the user's skin type by using a pre-learned skin type selection algorithm based on the obtained survey information.

The skin information providing device 100 according to an embodiment of the present disclosure may distinguish skin types according to oil and moisture and color and elasticity and select the user's skin type by using analysis information of survey information about key skin factors. For example, the key skin factors may include elasticity, skin tone, oil amount, moisture loss amount, nasolabial folds, or pores.

In operation S130, the skin information providing device 100 may analyze the distribution of the user's skin microbiome by using a pre-learned skin microbiome analysis algorithm based on the survey information.

The skin information providing device 100 according to an embodiment of the present disclosure may derive information about a plurality of microbiomes corresponding to the user's skin condition with respect to a skin microbiome distribution analyzed by age.

In operation S140, the skin information providing device 100 may provide information about the user's skin based on the survey information. The skin information providing device 100 may provide information about the user's skin based on the user's skin type and distribution of the user's skin microbiome.

The skin information providing device 100 according to an embodiment of the present disclosure may provide information about the mixing ratio of each microbiome with respect to a plurality of microbiomes corresponding to the user's skin condition.

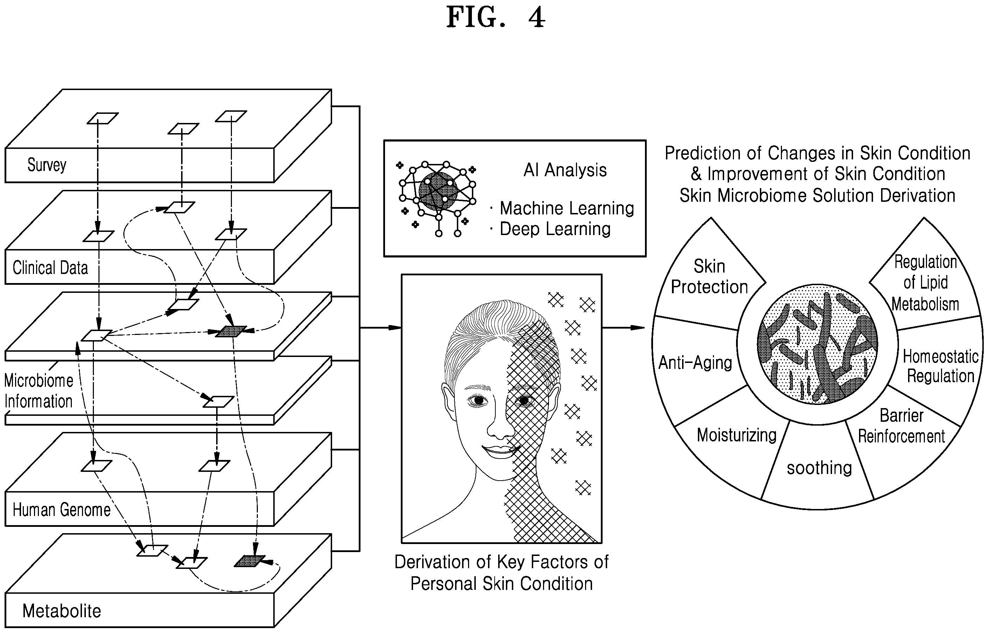

FIG. 4 is a diagram for explaining a method of providing skin information, according to an embodiment of the present disclosure.

Referring to FIG. 4, according to the method of providing skin information, according to an embodiment of the present disclosure, through an artificial intelligence (AI) algorithm, in which skin condition measurement information and microbiome analysis information on the skin surface, which are collected from about 1000 subjects from 0 years to 80 years of age, are combine, it is possible to measure the user's current skin condition and predict a skin condition that will change in the future, with just a brief survey filled out by the user using a user terminal. In addition, according to the method of providing skin information according to an embodiment of the present disclosure, it is possible to provide an optimal solution by not only analyzing and diagnosing the current condition of the skin, but also connecting skin care practices and products by applying microbiome materials to the skin condition.

The skin information providing device 100 according to an embodiment of the present disclosure may obtain survey information to provide skin information to the user. For example, as shown in FIG. 4, the skin information providing device 100 may obtain subjective survey information including the user's subjective response in relation to the user's skin condition. In addition, the skin information providing device 100 may obtain objective survey information including clinical data, microbiome data, human genome data, and/or metabolite data in relation to the user's skin condition. For example, the clinical data, the microbiome data, the human genome data, and/or the metabolome data may be in advance on a server. In addition, the skin information providing device 100 may obtain objective survey information related to clinical data, microbiome data, human genome data, and/or metabolite data.

The skin information providing device 100 according to an embodiment of the present disclosure may respond to the user's skin condition based on the subjective survey information and the objective survey information and derive key skin factors used in a skin type selection algorithm and a skin microbiome analysis algorithm.

The skin information providing device 100 according to an embodiment of the present disclosure may distinguish skin types according to oil and moisture and color and elasticity and select the user's skin type by using analysis information of survey information about key skin factors.

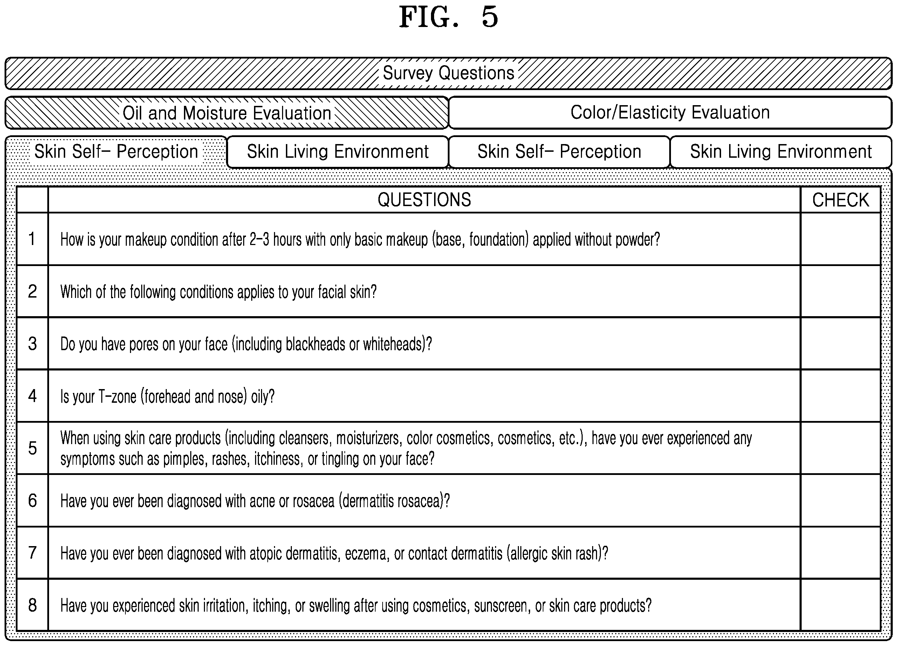

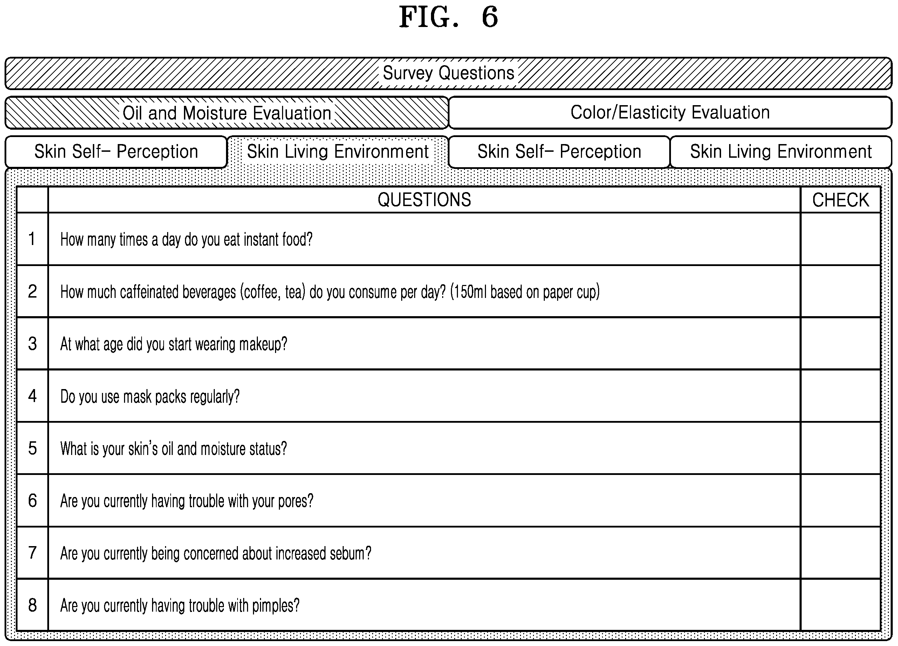

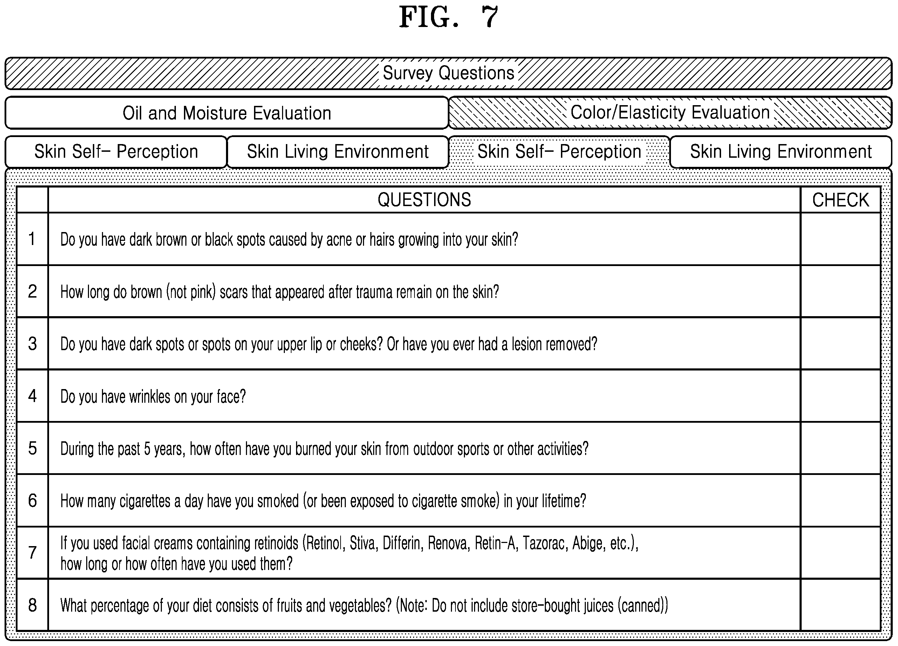

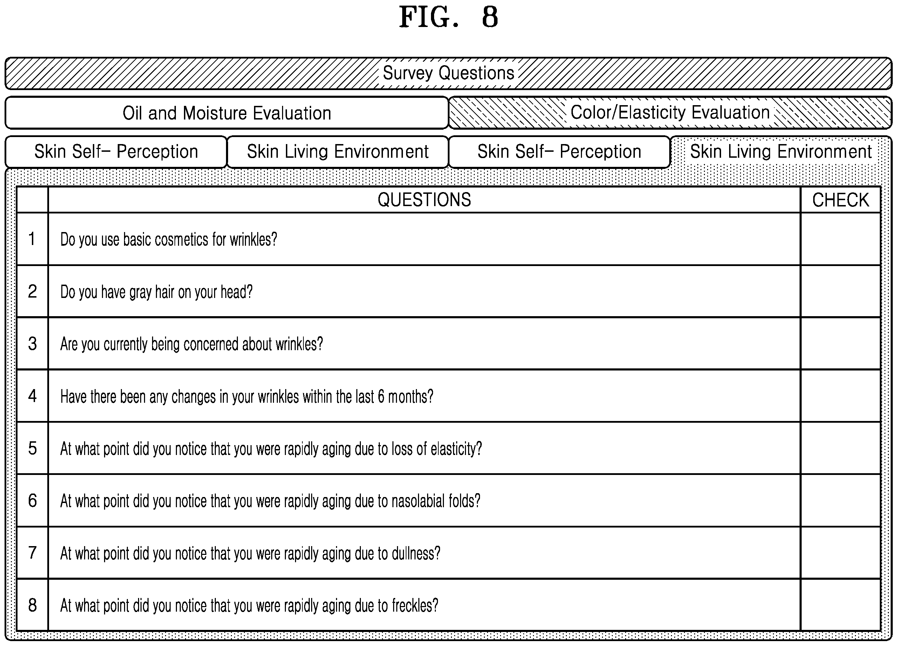

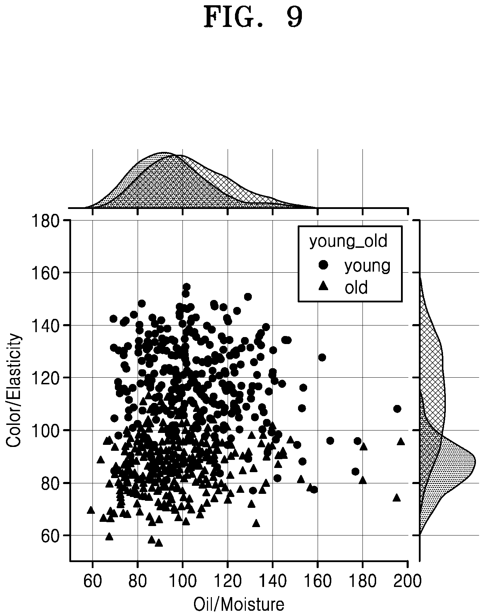

FIGS. 5 to 8 show examples of survey information related to the user's skin condition according to an embodiment of the present disclosure. In addition, FIG. 9 shows an example of key factors corresponding to the user's skin condition according to an embodiment of the present disclosure.

First, referring to FIGS. 5 to 8, examples of subjective survey information and objective survey information related to the user's skin condition are shown. For example, survey information related to the user's skin condition may be divided into items of skin self-perception and skin living environment with respect to oil and moisture evaluation and color elasticity evaluation.

For example, referring to the survey questions shown in FIGS. 5 to 8, the survey questions according to an embodiment of the present disclosure may include survey questions to obtain subjective survey information and objective survey information related to the user's skin condition based on clinical measurement data. For example, the survey questions may include questions about elasticity, skin tone, oil amount, moisture loss amount, nasolabial folds, pores, and the like, which have a positive or negative correlation with skin color, elasticity, oil, and moisture.

The skin information providing device 100 according to an embodiment of the present disclosure may obtain subjective survey information including the user's subjective response in relation to the user's skin condition. In addition, the skin information providing device 100 may obtain objective survey information including clinical data, microbiome data, human genome data, and/or metabolite data in relation to the user's skin condition. For example, as shown in FIG. 9, the skin information providing device 100 may obtain data based on big data analysis of about 1,000 Koreans in relation to the user's skin condition. For example, as shown in FIG. 9, the skin information providing device 100 may obtain data related to two factors, oil/moisture and color/elasticity, as key factors that significantly distinguish young/old groups. For example, the results shown in FIG. 9 may represent data obtained by analyzing the survey question data and clinical measurement data shown in FIGS. 5 to 8.

In addition, the skin information providing device 100 according to an embodiment of the present disclosure may derive key factors corresponding to the user's skin condition based on the subjective survey information and the objective survey information. Here, key factors may be used in a skin type selection algorithm and a skin microbiome analysis algorithm.

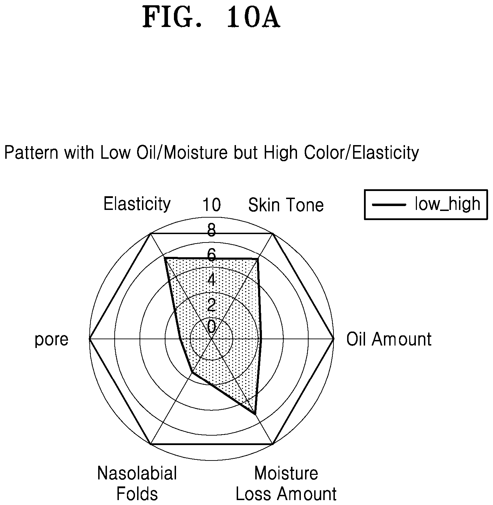

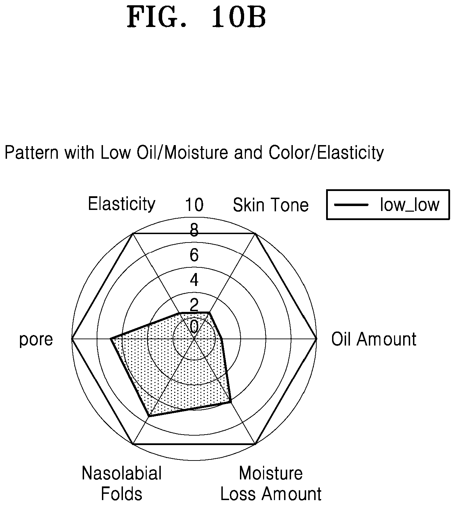

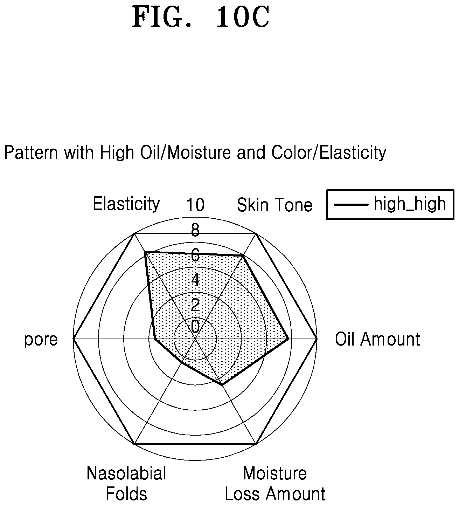

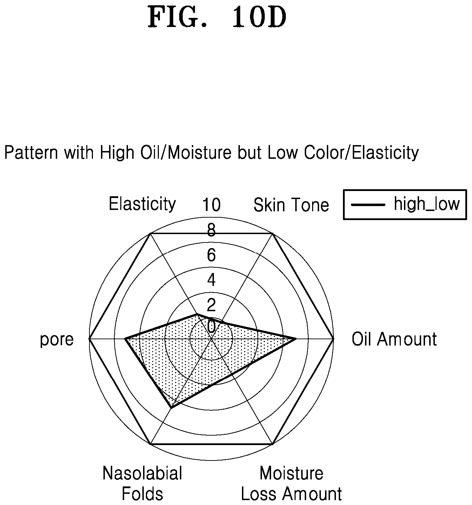

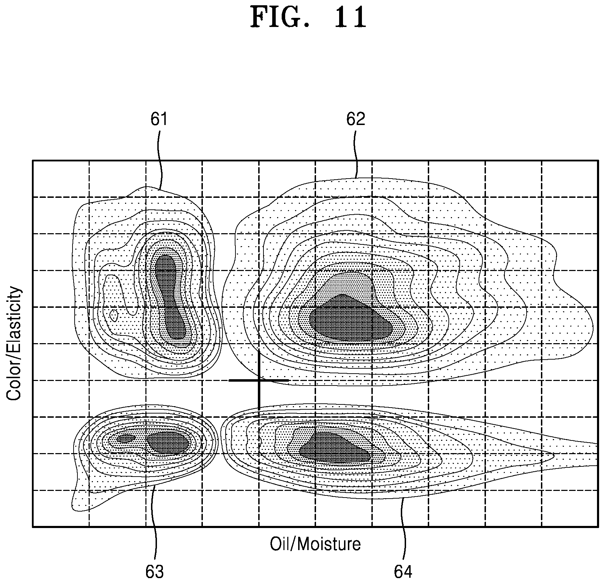

FIGS. 10A to 10D are diagrams for explaining a skin type selection process according to an embodiment of the present disclosure. In addition, FIG. 11 is a diagram for explaining a skin type selection process according to another embodiment of the present disclosure.

FIGS. 10A to 10D and 11, 4 skin types 61, 62, 63, and 64 selected according to oil/moisture and color/elasticity based on 6 factors (elasticity, skin tone, oil amount, moisture loss amount, nasolabial folds, and pores) are shown.

The skin information providing device 100 according to an embodiment of the present disclosure may give scores for each key skin factor based on survey information obtained from the user. In addition, the skin information providing device 100 may select 4 skin types by adding up the scores for each key skin factor that affects oil/moisture and color/elasticity. For example, the key skin factor may include elasticity, skin tone, oil amount, moisture loss amount, nasolabial folds, and pores.

The skin information providing device 100 may derive a skin type according to the user's survey input. That is, the skin information providing device 100 may weight key skin factors, in which subjective data and objective data included in the survey information match each other, and derive a skin type optimized for the user's current skin condition. For example, as shown in FIG. 11, the skin information providing device 100 may derive one skin type among four skin types 61, 62, 63, and 64 as a skin type optimized for the user's current skin condition.

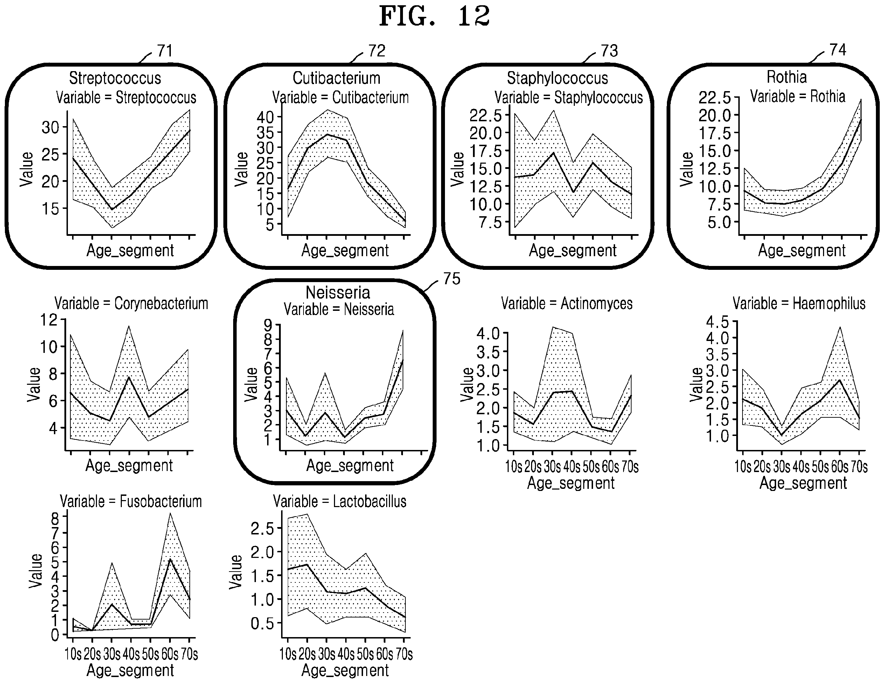

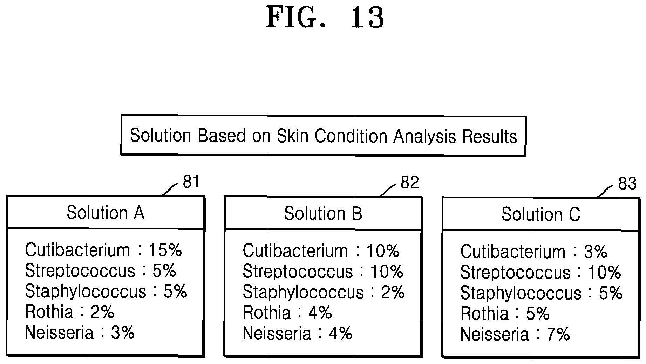

FIG. 12 is a diagram for explaining a microbiome analysis process according to an embodiment of the present disclosure. In addition, FIG. 13 is a diagram for explaining a method of providing skin information, according to another embodiment of the present disclosure.

First, referring to FIG. 12, five microbiomes secured according to skin microbiome distribution by age are shown. For example, a skin information providing device or server according to an embodiment of the present disclosure may store data on skin microbiome distribution by age in advance.

The skin information providing device 100 according to an embodiment of the present disclosure may secure a plurality of microbiomes corresponding to the skin condition of the user's age based on skin microbiome distribution data by age. For example, the skin information providing device 100 may secure at least one microbiome corresponding to the user's skin condition from among a plurality of microbiomes. For example, as shown in FIG. 12, when the user's age is in his 30s, the skin information providing device 100 may secure a first microbiome 71, a second microbiome 72, a third microbiome 73, a fourth microbiome 74, and a fifth microbiome 75, which are suitable for the skin condition of people in their 30s, based on age-specific skin microbiome distribution data for a plurality of microbiomes.

Referring to FIG. 13, the skin information providing device 100 according to an embodiment of the present disclosure may derive a skin type and microbiome result based on survey information obtained from the user. That is, the skin information providing device may weight key skin factors, in which subjective data and objective data included in the survey information match each other, and derive a skin type and a microbiome result, optimized for the user's current skin condition.

For example, as shown in FIGS. 12 and 13, the skin information providing device 100 according to an embodiment of the present disclosure may derive information about a plurality of microbiomes corresponding to the user's skin condition with respect to a skin microbiome distribution analyzed by age.

In addition, the skin information providing device 100 may provide information about the mixing ratio of each microbiome with respect to a plurality of microbiomes corresponding to the user's skin condition. For example, as shown in FIG. 13, the skin information providing device 100 according to an embodiment of the present disclosure may provide a first user with a first solution 81 according to the analysis of the first user's skin condition. In addition, the skin information providing device 100 may provide a second user with a second solution 82 according to the analysis of the second user's skin condition. In addition, the skin information providing device 100 may provide a third user with a third solution 83 according to the analysis of the third user's skin condition.

In addition, the skin information providing device 100 according to an embodiment of the present disclosure may recommend information about cosmetic products according to the mixing ratio of each microbiome to the user. For example, as shown in FIG. 13, the skin information providing device 100 may provide the first user with information about cosmetic products having a mixing ratio of each microbiome according to the first solution 81.

The algorithm or instructions described above may execute hardware, software, or program components, but is not limited to the above-described functions.

The 'algorithm' or the above-described 'Al algorithm' may be based on a previously input instruction or a modified and/or changed instruction. The modification and/or change may be due to a 'machine learning model', a 'deep learning model', or the like. The machine learning model may include any model used to infer an answer to a given input. Here, the machine learning model may be trained to infer annotation information for target data. For example, a hint-based machine learning model may be trained to infer annotation information about skin information and/or information related thereto by using hint information. Annotation information generated through an annotation work may be used as hint information to train a machine learning model. In addition, the machine learning model may include weights associated with a plurality of nodes included in the machine learning model. Here, the weights may include arbitrary parameters associated with the machine learning model. The deep learning model may be configured to output an answer to an input value and explain or show an interpretation of the output answer, and may include an interpretable model for an inferred answer. As a result, the 'algorithm' or the above-described 'Al algorithm' may be performed by a previously input instruction, or may be performed by an instruction modified and/or changed by the 'algorithm' or 'Al algorithm'.

The 'training' may refer to any process of changing weights associated with a machine learning model by using target data and annotation information. According to an embodiment, the hint-based machine learning model may be trained to infer a plurality of annotation information items associated with a plurality of target data items by using hint information including a plurality of pixel groups and a plurality of annotation classes, which are extracted from target data.

The 'annotation information' may be information obtained as a result of annotation work as correct answer information of a data sample. Annotations or annotation information may be used interchangeably with terms, such as labels and tags, in the relevant technical field.

The module and/or unit described above may be implemented with a processor and a memory. The 'processor' has to be broadly interpreted to include general-purpose processors, central processing units (CPUs), microprocessors, digital signal processors (DSPs), controllers, microcontrollers, state machines, and the like. In some circumstances, the 'processor' may refer to an application-specific integrated circuit (ASIC), a programmable logic device (PLD), a field programmable gate array (FPGA), or the like. The processor may refer to a combination of processing devices, for example, a combination of a DSP and a microprocessor, a combination of a plurality of microprocessors, a combination of one or more microprocessors in combination with a DSP core, or a combination of any other configurations. In addition, the above-described memory has to be broadly interpreted to include any electronic component capable of storing electronic information. The memory may refer to various types of processor-readable media, such as random access memory (RAM), read-only memory (ROM), non-volatile random access memory (NVRAM), programmable read-only memory (PROM), erasable-programmable read-only memory (EPROM), electrically erasable PROM (EEPROM), flash memory, magnetic or optical data storage, and registers. When the processor may read information from and/or write information to the memory, the memory may be said to be in electronic communication with the processor. The memory integrated into the processor may be in electronic communication with the processor.

The devices and/or systems described above may be implemented as hardware components, software components, and/or a combination of hardware components and software components. The devices and components described in the embodiments may be, for example, implemented by using one or more implemented using one or more general-purpose computers or special-purpose computers, such as an arithmetic logic unit (ALU), a digital signal processor, a field programmable gate array (FPGA), a programmable logic unit (PLU), a microprocessor, or any other device that may execute and respond to instructions. A processing device may run an operating system (OS) and one or more software applications running on the operating system. Also, the processing device may also access, store, manipulate, process, and generate data in response to execution of software. For convenience of understanding, a case where one processing unit is used is described, but those skilled in the art will appreciate that a processing device may include multiple processing elements and/or a plurality of types of processing elements. For example, the processing device may include a plurality of processors or a processor and a controller. Also, other processing configurations, such as parallel processors, may also be made.

Software may include a computer program, code, instructions, or a combination of one or more thereof, which may configure a processing device to operate as desired or may command the processing devices independently or collectively. Software and/or data may be permanently or temporarily embodied in any tangible machine, component, physical device, virtual equipment, computer storage medium or device, or transmitted signal wave to be interpreted by a processing device or to provide instructions or data to the processing device. Software may be distributed on networked computer systems and stored or executed in a distributed manner. Software and data may be stored on one or more computer-readable recording media.

The system described above may include at least one of a server device and a cloud device, but is not limited thereto. For example, the system may consist of one or more server devices. As another example, the system may consist of one or more cloud devices. As another example, the system may be operated with a server device and a cloud device configured together.

The method according to an embodiment may be implemented in the form of program instructions that can be executed through various computer units and recorded on a computer readable recording medium. A computer-readable recording medium may include program instructions, data files, data structures, etc. alone or in combination. Program instructions recorded on the medium may be specially designed and configured for the embodiments or may be known and usable to those skilled in computer software. Examples of computer-readable recording media include hardware devices specially configured to store and execute program instructions, such as magnetic media such as hard disks, floppy disks, and magnetic tapes, optical media such as CD-ROMs and DVDs, magneto-optical media such as floptical disks, and ROM, RAM, and flash memory. Examples of program instructions include high-level language codes that can be executed by a computer using an interpreter or the like, as well as machine language codes such as those produced by a compiler. The hardware devices described above may be configured to operate as one or more software modules to perform the operations of the embodiments, and vice versa.

As such, the present disclosure has been described with reference to the embodiments shown in the drawings, but these are merely illustrative, and it will be understood by those of ordinary skill in the art that various modifications and equivalent other embodiments may be made therefrom. Therefore, the scope of technical protection of the present disclosure should be determined by the technical spirit of the attached claims.

## Claims

1. A device for providing skin information, the device comprising:
a communication unit forming a network connection with an external device;
a user interface unit;
a processor; and
a memory storing instructions executable by the processor,
wherein the processor is configured to, by executing the instructions,
obtain survey information related to a user's skin condition and, based on the survey information, select the user's skin type, analyze the user's skin microbiome distribution, or provide information about the user's skin.

2. The device of claim 1, wherein
the processor is further configured to, by executing the instructions,
obtain subjective survey information including the user's subjective response in relation to the user's skin condition and
objective survey information including clinical data, microbiome data, human genome data, or metabolite data in relation to the user's skin condition.

3. The device of claim 2, wherein
the processor is further configured to, by executing the instructions,
derive key skin factors based on the subjective survey information and the objective survey information, the key skin factors corresponding to the user's skin condition and being used in a skin type selection algorithm and a skin microbiome analysis algorithm.

4. The device of claim 1, wherein
the processor is further configured to, by executing the instructions,
distinguish skin types according to oil, moisture, color, and elasticity and select the user's skin type by using analysis information of the survey information about key skin factors.

5. The device of claim 4, wherein
the key skin factors comprise elasticity, skin tone, oil amount, moisture loss amount, nasolabial folds, or pores.

6. The device of claim 1, wherein
the processor is further configured to, by executing the instructions,
derive information about a plurality of microbiomes corresponding to the user's skin condition with respect to a skin microbiome distribution analyzed by age.

7. The device of claim 6, wherein
the processor is further configured to, by executing the instructions,
provide information about a mixing ratio of each microbiome with respect to a plurality of microbiomes corresponding to the user's skin condition.

8. The device of claim 1, wherein
the processor is further configured to, by executing the instructions,
select the user's skin type by using a pre-learned skin type selection algorithm.

9. The device of claim 1, wherein
the processor is further configured to, by executing the instructions,
analyze the user's skin microbiome distribution by using a pre-learned skin microbiome analysis algorithm based on the survey information.

10. The device of claim 1, wherein
the processor is further configured to, by executing the instructions,
provide information about the user's skin based on the user's skin type or the user's skin microbiome distribution.

11. A method of providing skin information, the method comprising:
obtaining survey information related to a user's skin condition; and
selecting the user's skin type by using a pre-learned skin type selection algorithm based on the survey information, analyzing the user's skin microbiome distribution by using a pre-learned skin microbiome analysis algorithm based on the survey information, or providing information about the user's skin based on the survey information.

12. The method of claim 11, wherein
the obtaining of the survey information comprises obtaining subjective survey information including the user's subjective response in relation to the user's skin condition and
objective survey information including clinical data, microbiome data, human genome data, or metabolite data in relation to the user's skin condition.

13. The method of claim 12, wherein
the obtaining of the survey information comprises deriving key skin factors based on the subjective survey information and the objective survey information, the key skin factors corresponding to the user's skin condition and being used in the skin type selection algorithm and the skin microbiome analysis algorithm.

14. The method of claim 11, wherein
the selecting of the user's skin type comprises distinguishing skin types according to oil, moisture, color, and elasticity and selecting the user's skin type by using analysis information of the survey information about key skin factors.

15. The method of claim 14, wherein
the key skin factors comprise elasticity, skin tone, oil amount, moisture loss amount, nasolabial folds, or pores.

16. The method of claim 11, wherein
the analyzing of the user's skin microbiome distribution comprises deriving information about a plurality of microbiomes corresponding to the user's skin condition with respect to a skin microbiome distribution analyzed by age.

17. The method of claim 16, wherein
the providing of the information about the user's skin comprises providing information about a mixing ratio of each microbiome with respect to a plurality of microbiomes corresponding to the user's skin condition.

18. The method of claim 11, wherein
the providing of the information about the user's skin comprises providing information about the user's skin based on the user's skin type or the user's skin microbiome distribution.

19. A computer program stored in a computer-readable recording medium to execute the method of claim 11 by using a computing device.
